# EUROPÄISCHE PATENTSCHRIFT

(11) **EP 0 708 634 B1**
(45) Veröffentlichungstag und Bekanntmachung des Hinweises auf die Patenterteilung: **03.09.1997**
(21) Anmeldenummer: 94919666.1
(22) Anmeldetag: 28.06.1994
(51) Int. Cl.: A61K 7/13

(54) **FÄRBEMITTEL MIT TETRAAMINOPYRIMIDIN UND DIREKTZIEHERN**
DYES CONTAINING TETRAAMINOPYRIMIDINE AND DIRECT-DYEING AGENTS
PRODUITS COLORANTS CONTENANT DE LA TETRA-AMINOPYRIMIDINE ET DES COLORANTS MONTANT DIRECTEMENT SUR LA FIBRE

(30) Priorität: 07.07.1993 DE 4322541
(43) Veröffentlichungstag der Anmeldung: 01.05.1996
(73) Patentinhaber: Henkel Kommanditgesellschaft auf Aktien, 40191 Düsseldorf (DE)
(72) Erfinder: MÜLLER, Reinhard, D-41812 Erkelenz (DE); HÖFFKES, Horst, D-40595 Düsseldorf (DE); HOLLENBERG, Detlef, D-40699 Erkrath (DE); SCHRAMM, Christa, D-40468 Düsseldorf (DE)
(86) Internationale Anmeldenummer: EP9402100
(87) Internationale Veröffentlichungsnummer: WO9501713

(56) Entgegenhaltungen:
- FR-A- 2 135 138
- US-A- 5 037 446
- US-E- 30 199

## Beschreibung

Die Erfindung betrifft Mittel zum Färben von keratinhaltigen Fasern, die neben üblichen Kupplerkomponenten 2,4,5,6-Tetraaminopyrimidine und bestimmte direktziehende Farbstoffe enthalten.

Für das Färben von keratinhaltigen Fasern, z. B. von Haaren, Pelzen, Fellen und Wolle spielen die sogenannten Oxidationshaarfärbemittel wegen ihrer intensiven Farben und guten Echtheitseigenschaften eine bevorzugte Rolle.

Solche Haarfärbemittel enthalten Oxidationsfarbstoffvorprodukte in einem wäßrigen Träger. Als Oxidationsfarbstoffvorprodukte werden Entwicklerkomponenten und Kupplerkomponenten eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen folgende Voraussetzungen erfüllen: Sie müssen bei der oxidativen Kupplung die gewünschten Nuancen in ausreichender Farbstärke und Echtheit ausbilden. Bei ihrer Anwendung in Haarfärbemitteln sollen sie bereits bei niedrigen Temperaturen unterhalb 40 °C ein gutes Aufziehvermögen auf menschlichem Haar besitzen, wobei keine merklichen Unterschiede zwischen strapaziertem und frisch nachgewachsenem Haar bestehen dürfen. Diese Eigenschaft wird auch als Egalisiervermögen bezeichnet.

Die erzeugten Haarfärbungen sollen weiterhin beständig sein gegen Licht, Wärme, mechanische Beanspruchung (Reibechtheit der Anfärbung), Beanspruchung durch Haarwaschmittel (Waschechtheit der Anfärbung) und dem Einfluß chemischer Reduktionsmittel, z. B. Dauerwellflüssigkeiten. Schließlich soll die Kopfhaut während des Färbevorgangs nicht zu sehr angefärbt werden und vor allem sollen die verwendeten Substanzen in toxikologischer und dermatologischer Hinsicht unbedenklich sein.

Als Entwicklerkomponenten werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate und 4-Aminopyrazolonderivate eingesetzt.

Übliche Kupplerkomponenten sind Meta-Phenylendiaminderivate, Dihydroxynaphthaline, Naphthole, Resorcinderivate, Meta-Aminophenole und Pyrazolone, z. B. α-Naphthol, 1,5-Dihydroxynaphthalin, 1,7-Dihydroxynaphthalin, 2,7-Dihydroxynaphthalin, 5-Amino-2-Methylphenol, m-Aminophenol, Resorcin, Resorcinmonomethylether, Meta-Phenylendiamin, 1-Phenyl-3-Methylpyrazolon-5, 2,4-Dichlor-3-aminophenol, 1,3-Bis-(2,4-diaminophenoxy)-propan, 4-Chlorresorcin, 2-Chlor-3-Amino-6-Methylphenol, 2-Methylresorcin, 2,4-Diaminophenol.

Aus den deutschen Offenlegungsschriften DE-OS-27 14 831, DE-OS-27 16 475 und DE-OS-27 17 041 ist die Verwendung von Tetraaminopyrimidinen als Entwicklerkomponente in Haarfärbemitteln in Kombination mit bestimmten Kupplerkomponenten bekannt.

Hinsichtlich des Egalisiervermögen sind die aus dem Stand der Technik bekannten Färbemittel auf Basis von 2,4,5,6-Tetraaminopyrimidinen jedoch nicht voll zufriedenstellend. Außerdem läßt die Waschechtheit der erzielten Färbungen zu wünschen übrig.

Es stellt sich deshalb die Aufgabe, Färbemittel auf Basis von 2,4,5,6-Tetraaminopyrimidinen bereitzustellen, die über ein verbessertes Egalsiervermögen verfügen und die Ausfärbungen mit guten Waschechtheiten ergeben.

Überraschenderweise wurde nun gefunden, daß durch den Zusatz geringer Mengen an bestimmten direktziehenden Farbstoffen zu Färbemitteln auf Basis von 2,4,5,6-Tetraminopyrimidinen Egalisiervermögen und Waschechtheit verbessert werden können.

Gegenstand der Erfindung sind Mittel zum Färben von keratinhaltigen Fasern enthaltend als Entwicklerkomponente mindestens ein 2,4,5,6-Tetraaminopyrimidin der Formel I 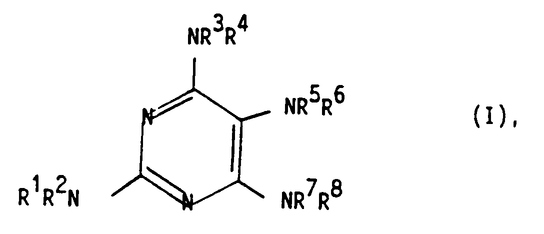 worin R¹, R², R³, R⁴, R⁵, R⁶, R⁷ und R⁸ unabhängig voneinander Wasserstoff, C₁-C₄-Alkylgruppen oder C₂-C₄-Hydroxyalkylgruppen bedeuten,
mindestens eine übliche Kupplerkomponente und einen wasserhaltigen Träger, dadurch gekennzeichnet, daß ein direktziehender Farbstoff der Formel II 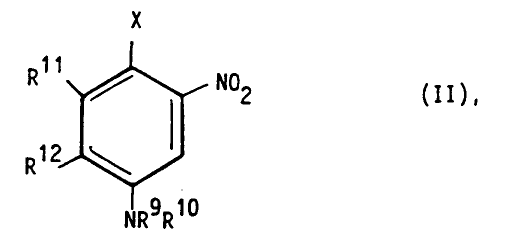 worin R⁹ und R¹⁰ unabhängig voneinander Wasserstoff, C₁-C₄-Alkylgruppen oder Hydroxy- oder Dihydroxy-C₂-C₄-alkylgruppen bedeuten,
R¹¹ und R¹² Wasserstoff, Chlor oder Hydroxy darstellen, mit der Maßgabe, daß R¹¹ Chlor ist, wenn R¹² Wasserstoff oder Hydroxy ist und daß R¹¹ Wasserstoff oder Hydroxy ist, wenn R¹² Chlor ist, und
X Wasserstoff oder eine Gruppe NR¹³R¹⁴ darstellt, wobei R¹³ und R¹⁴ unabhängig voneinander Wasserstoff, C₁-C₄-Alkylgruppen oder Hydroxy- oder Dihydroxy-C₂-C₄-alkylgruppen darstellen,
oder
ein direktziehender Farbstoff der Formel III 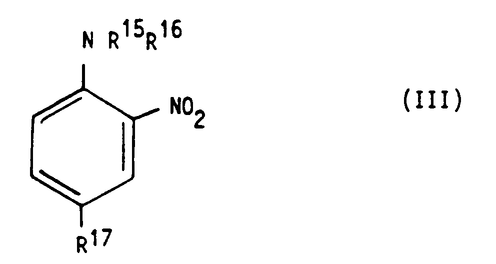 worin R¹⁵ und R¹⁶ unabhängig voneinander Wasserstoff, C₁-C₄-Alkylgruppen oder Hydroxy- oder Dihydroxy-C₂-C₄-alkylgruppen bedeuten und R¹⁷ eine NH₂-Gruppe, eine C₁-C₄-Alkylgruppe oder eine Hydroxy- oder Dihydroxy-C₂-C₄-alkylgruppe darstellt,
enthalten ist.

In einer erfindungsgemäßen Färbemittelzubereitung können auch verschiedene Verbindungen der Formeln I, II oder III zum Einsatz kommen.

Eine Färbemittelzubereitung kann auch eine Verbindung der Formel II und eine Verbindung der Formel III gemeinsam enthalten, desweiteren können andere übliche direktziehende Farbstoffe, z. B. Nitrophenylendiaminderivate, Antrachinonderivate oder Naphthochinonderivate, enthalten sein.

In den erfindungsgemäßen Färbemitteln können weiterhin andere übliche Entwicklerkomponenten enthalten sein.

Zur Ausbildung natürlich wirkender Farbnuancen werden bevorzugt auch Kupplerverbindungen eingesetzt, die bei der Oxidation den von der Entwicklerkomponente der Formel I erzeugten Farbton modifizieren. Geeignete Kuppler wurden bereits oben genannt.

Alle in den Färbemitteln enthaltenden 2,4,5,6-Tetraaminopyrimidine der Formel I, Kupplerkomponenten, Farbstoffe der Formel II oder III und gegebenenfalls weitere übliche Entwicklerkomponenten und direktziehende Farbstoffe können auch in Form ihrer wasserlöslichen Salze, z. B. der Hydrochloride, Hydrobromide oder Sulfate vorliegen, sofern sie zur Salzbildung befähigt sind.

Vorzugsweise enthalten die erfindungsgemäßen Färbemittel als Entwicklerkomponente der Formel I unsubstituiertes 2,4,5,6,-Tetraaminopyrimidin.

Besonders bevorzugt sind erfindungsgemäße Färbemittel in denen der Farbstoff der Formel II ausgewählt ist aus 6-Chlor-4-nitro-2-aminophenol, 5-Chlor-4-(2-hydroxyethyl)amino-2-nitroanilin, 5-Chlor-4-(2,3-dihydroxypropyl)amino-2-nitroanilin und 1,4-Bis-[(2,3-dihydroxypropyl)-amino]-5-chlor-2-nitrobenzol und in denen der Farbstoff der Formel III 1-(2-Hydro xyethyl)amino-4-methyl-2-nitrobenzol ist.

Die erfindungsgemäßen Färbemittel ergeben schon bei physiologisch verträglichen Temperaturen von unter 40 °C intensive Färbungen. Sie eignen sich deshalb besonders zum Färben von menschlichen Haaren. Ein weiterer Erfindungsgegenstand sind Haarfärbemittel enthaltend
- 2,4,5,6-Tetraaminopyrimidin in einer Menge von 0,01 bis 5 Gew.-%, vorzugsweise 0,1 bis 1 Gew.-%,
- übliche Kupplerkomponenten in einer Menge von 0,01 bis 5 Gew.-%, vorzugsweise 0,1 bis 1 Gew.-%,
- mindestens einen direktziehenden Farbstoff ausgewählt aus der Gruppe 6-Chlor-4-nitro-2-aminophenol, 5-Chlor-4-(2-hydroxyethyl)amino-2-nitroanilin, 5-Chlor-4-(2,3-dihydroxypropyl)amino-2-nitroanilin und 1,4-Bis-[(2,3-dihydroxypropyl)-amino]-5-chlor-2-nitrobenzol in einer Gesamtmenge von 0,005 bis 1 Gew.-%, vorzugsweise 0,01 bis 0,3 Gew.-%,
   wobei alle Gew.-% Angaben bezogen sind auf die gesamte Haarfärbemittelzubereitung,
- und einen wasserhaltigen Träger.

Ein weiterer Erfindungsgegenstand sind Haarfärbemittel enthaltend
- 2,4,5,6-Tetraaminopyrimidin in einer Menge von 0,01 bis 5 Gew.-%, vorzugsweise 0,1 bis 1 Gew.-%,
- übliche Kupplerkomponenten in einer Menge von 0,01 bis 5 Gew.-%, vorzugsweise 0,1 bis 1 Gew.-%,
- 1-(2-Hydroxyethyl)amino-4-methyl-2-nitrobenzol in einer Menge von 0,005 bis 1 Gew.-%, vorzugsweise 0,01 bis 0,3 Gew.-%,
   wobei alle Gew.-% Angaben bezogen sind auf die gesamte Haarfärbemittelzubereitung,
- und einen wasserhaltigen Träger.

Geeignete wasserhaltige Träger sind z. B. Cremes, Emulsionen, Gele oder auch tensidhaltige schäumende Lösungen, wie z. B. Shampoos oder andere Zubereitungen, die für die Anwendung auf dem Haar geeignet sind. Der wasserhaltige Träger enthält üblicherweise Netz- und Emulgiermittel wie anionische, nichtionische oder ampholytische Tenside, z. B. Fettalkoholsulfate, Alkansulfonate, α-Olefinsulfonate, Fettalkoholpolyglykolethersulfate, Alkylglykoside, Ethylenoxidanlagerungsprodukte an Fettalkohole, an Alkylphenole, an Fettsäuren, an Sorbitanfettsäureester, an Fettsäurepartialglyceride und Fettsäurealkanolamide; Verdickungsmittel z. B. Fettalkohole, Fettsäuren, Fettsäureester und andere Fettkomponenten in emulgierter Form; wasserlösliche polymere Verdickungsmittel wie natürliche Gummen, z. B. Gummiarabicum, Karagummi, Guargummi, Johannisbrotkernmehl, Leinsamengummen und Pektin, biosynthetische Gummen, z. B. Xanthangummi und Dextrane, synthetische Gummen, z. B. Agar-Agar und Algin, Stärkefraktionen und Derivate, wie Amylose, Amylopektin und Dextrine, modifizierte Cellulosemoleküle, z. B. Methylcellulose, Hydroxyalkylcellulose und Carboxymethylcellulose, Tone, wie z. B. Bentonit oder vollsynthetische Hydrokolloide, wie z. B. Polyvinylalkohol oder Polyvinylpyrrolidon, haarpflegende Zusätze wie z. B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfümöle sowie Reduktionsmittel zur Stabilisierung der Inhaltsstoffe, z. B. Ascorbinsäure; schließlich können auch Farbstoffe zum Einfärben der Zubereitungen enthalten sein.

Bei Zusatz der o. g. direktziehenden Farbstoffe der Formel II bzw. III in den genannten Mengen beobachtet man überraschenderweise eine Verbesserung des Egalisiervermögens sowohl gegenüber Färbemitteln, die die o. g. direktziehenden Farbstoffe der Formel II bzw. III nicht enthalten, als auch gegenüber Färbemitteln, die diese direktziehenden Farbstoffe in einer Gesamtmenge von über 1 Gew.-% enthalten.

Auch hinsichtlich der Waschechtheiten sind die erzielten Ausfärbungen dann am besten, wenn der Gehalt an den o. g. direktziehenden Farbstoffen der Formel II bzw. III im erfindungsgemäßen Bereich von 0,005 bis 1 Gew.-%, vorzugsweise 0,01 bis 0,3 Gew.-%, liegt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein aufhellender Effekt am Haar gewünscht wird. Als Oxidationsmittel kommen insbesondere Wasserstoffperoxid oder dessen Anlagerungsprodukte an Harnstoff, Melamin oder Natriumborat in Betracht.

Bevorzugt wird eine Zubereitung des Oxidationsmittels unmittelbar vor dem Haarefärben mit der Zubereitung aus Oxidationsfarbstoffvorprodukten und Träger vermischt. Das dabei entstehende gebrauchsfertige Haarfärbepräparat sollte bevorzugt einen pH-Wert von 6 bis 10 aufweisen. Besonders bevorzugt ist die Anwendung der Haarfärbemittel in einem schwach-alkalischen Milieu. Die Anwendungstemperaturen können in einem Bereich von zwischen 15 und 40 °C liegen. Nach einer Einwirkungszeit von ca. 30 Minuten wird das Haarfärbemittel durch Auspülen von dem zu färbenden Haar entfernt. Das Nachwaschen mit einem Shampoo entfällt, wenn ein stark tensidhaltiger Träger z. B. ein Färbeshampoo verwendet wurde.

Die folgenden Beispiele sollen den Erfindungsgegenstand näher erläutern, ohne ihn jedoch hierauf zu beschränken.

### Beispiele

Es wurden die Färbemittel 1, 2 und 3 in Form einer Creme-Emulsion der folgenden Zusammensetzungen hergestellt (die Färbemittel 2 und 3 sind erfindungsgemäß, Färbemittel 1 dient zum Vergleich).

| | 1 | 2 | 3 |
|---|---|---|---|
| C₁₂-C₁₈-Fettalkohol | 10 g | 10 g | 10 g |
| C₁₂-C₁₄-Fettalkohol + 2 EO-Sulfat, Natriumsalz (28 %ig) | 25 g | 25 g | 25 g |
| Wasser | 60 g | 60 g | 60 g |
| 2,7-Dihydroxynaphthalin | 0,076 g | 0,076 g | 0,076 g |
| p-Toluylendiamin | 0,093 g | 0,093 g | 0,093 g |
| 2-Methyl-1,3-dihydroxybenzol | 0,036 g | 0,036 g | 0,036 g |
| 2,4,5,6-Tetraaminopyrimidin | 0,172 g | 0,172 g | 0,172 g |
| 6-Chlor-4-nitro-2-aminophenol | - | 0,05 g | - |
| 1-(2-Hydroxyethyl)amino-4-methyl-2-nitrobenzol | - | - | 0,05 g |
| Ascorbinsäure (Inhibitor) | 0,4 g | 0,4 g | 0,4 g |
| Na₂SO₃ (Inhibitor) | 0,5 g | 0,5 g | 0,5 g |
| (NH₄)₂SO₄ | 1,0 g | 1,0 g | 1,0 g |
| Konz. NH₃-Lösung | bis pH=9,5 | bis pH=9,5 | bis pH=9,5 |
| Wasser | ad 100 g | ad 100 g | ad 100 g |

Die oxidative Entwicklung der Färbung wurde mit 6 %iger Wasserstoffperoxidlösung als Oxidationslösung durchgeführt. Hierzu wurden 50 g der Emulsion mit 50 g Wasserstoffperoxidlösung (6 %ig) versetzt und vermischt.

Die Färbecreme wurde auf ca. 5 cm lange Strähnen standardisierten zu 90 % ergrautem aber nicht besonders vorbehandeltem Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet. Die Färbemittel 1, 2 und 3 ergaben eine gold-blonde Nuance.

In gleicher Weise wurden Egalisiersträhnen gefärbt; Egalisiersträhnen wurden wie folgt hergestellt: Die obere Hälfte der Haarsträhne (der Bereich der Haarspitze) wurde 30 Minuten lang mit der wäßrigen Lösung eines Kaltwellmittels auf Basis Ammoniumthioglycolat behandelt. Nach der Fixierung (10 Minuten, Kaliumbromatlösung) wurde die gleiche Hälfte der Haarsträhne mit einer wäßrigen Lösung aus Wasserstoffperoxid und Ammoniumperoxiddisulfat ultrablondiert. Anschließend erfolgte noch einmal eine Behandlung mit dem Kaltwellmittel, der Fixierung und der Ultrablondierung. Die untere Hälfte der Haarsträhne (Wurzelbereich) wurde nur einmal ultrablondiert. Auf diese Weise wurden zwei unterschiedlich stark strapazierte Haarsträhnenhälften erhalten.

### Tabelle 1:

### Waschechtheit der mit den Färbemitteln 1, 2 und 3 erzielten Färbungen

Die Farbintensitäten des geschädigten und des ungeschädigten Teils einer gefärbten, ungewaschenen Egalisiersträhne wurden farbmetrisch bestimmt und jeweils zu 100 % gesetzt.

Nach 6-maligem Waschen mit einem üblichen Haarshampoo wurden die Farbintensitäten der geschädigten und die Farbintensitäten der ungeschädigten Teile nochmals gemessen und ins Verhältnis zu den jeweiligen Farbintensitäten der ungewaschenen Strähne gesetzt. Daraus ergeben sich die Werte für die Waschechtheiten (Angaben in %).

**Tabelle 1**

| | 1 | 2 | 3 |
|---|---|---|---|
| geschädigter Teil der Egalisiersträhne | 45 % | 50 % | 51 % |
| ungeschädigter Teil der Egalisiersträhne | 53 % | 57 % | 56 % |

Es ist erkennbar, daß bei Verwendung der erfindungsgemäßen Färbemittel 2 und 3 nach 6-maligem Waschen eine größere Restfarbintensität auf den Strähnen verbleibt als bei Verwendung des nicht erfindungsgemäßen Vergleichs-Färbemittels 1. Dies gilt sowohl für den geschädigten als auch den ungeschädigten Teil der Egalisiersträhnen.

Analoge Versuche mit Färbemitteln, die als Farbstoffe die direktziehenden Farbstoffe 6-Chlor-4-nitro-2-aminophenol bzw. 1-(2-Hydroxyethyl)amino-4-methyl-2-nitrobenzol alleine in einer Menge von 0,05 Gew.-% enthielten, ergaben, daß nach 6-maligem Waschen die Restfarbintensitäten sowohl auf dem geschädigten als auch auf dem ungeschädigten Teil der Egalisiersträhne praktisch Null sind.

### Tabelle 2:

### Egalisiervermögen der Färbemittel 1, 2 und 3

Die Farbintensitäten des geschädigten und des ungeschädigten Teils einer gefärbten, 6-mal gewaschenen Egalisiersträhne wurden gemessen.

Dann wurde die Farbintensität des ungeschädigten Teils ins Verhältnis zur Farbintensität des geschädigten Teils gesetzt. Daraus ergeben sich die Werte für das Egalisiervermögen an einer 6-mal gewaschenen Egalisiersträhne (Angaben in %):

**Tabelle 2**

| 1 | 2 | 3 |
|---|---|---|
| 57 % | 76 % | 74 % |

Es ist erkennbar, daß bei Verwendung der erfindungsgemäßen Färbemittel 2 und 3 das Verhältnis der Farbintensität des ungeschädigten Teils der Egalisiersträhne zur Farbintensität des geschädigten Teils der Egalisiersträhne näher am Idealwert 100 % liegt als bei Verwendung des nicht erfindungsgemäßen Färbemittels 1.

Es wurden weitere Färbemittel 4, 5 und 6 in Form einer Creme-Emulsion analog zu den Färbemitteln 1, 2 und 3 hergestellt, jedoch mit anderen Oxidationsfarbstoffvorprodukten und Direktziehern (die Färbemittel 5 und 6 sind erfindungsgemäß, Färbemittel 4 dient zum Vergleich):

| | 4 | 5 | 6 |
|---|---|---|---|
| 2-Chlor-3-amino-6-methylphenol x HCl | 0,194 g | 0,194 g | 0,194 g |
| p-Toluylendiamin | 0,220 g | 0,220 g | 0,220 g |
| 2,4,5,6-Tetraaminopyrimidin | 0,130 g | 0,130 g | 0,130 g |
| 6-Chlor-4(2-hydroxyethyl)amino-2-nitroanilin | - | 0,30 g | - |
| Gemisch aus 5-Chlor-4-(2,3-dihydroxypropylamino)-2-nitroanilin und 1,4-Bis-[(2,3-dihydroxypropyl)-amino]-5-chlor-2-nitrobenzol | - | - | 0,30 g |

Die Ausfärbung wurde analog zu den Beispielen 1, 2 und 3 durchgeführt. Es resultierte eine auberginefarbene Nuance.

### Tabelle 3:

### Egalisiervermögen der Färbemittel 4, 5 und 6

Die Messungen wurden wie oben beschrieben durchgeführt.

Egalsisiervermögen (Angaben in %):

**Tabelle 3**

| 4 | 5 | 6 |
|---|---|---|
| 59 % | 80 % | 81 % |

Es zeigt sich, daß bei Verwendung der erfindungsgemäßen Färbemittel 5 und 6 das Verhältnis der Farbintensität des ungeschädigten Teils der Egalisiersträhne zur Farbintensität des geschädigten Teils der Egalisiersträhne näher am Idealwert 100 % liegt als bei Verwendung des nicht erfindungsgemäßen Färbemittels 4.

## Patentansprüche

1. Mittel zum Färben von keratinhaltigen Fasern enthaltend als Entwicklerkomponente mindestens
ein 2,4,5,6-Tetraaminopyrimidin der Formel I 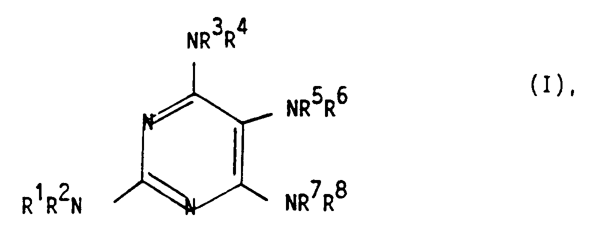 worin R¹, R², R³, R⁴, R⁵, R⁶, R⁷, R⁸ unabhängig voneinander Wasserstoff, C₁-C₄-Alkylgruppen oder C₂-C₄-Hydroxyalkylgruppen bedeuten,
mindestens eine übliche Kupplerkomponente und einen wasserhaltigen Träger,
dadurch gekennzeichnet, daß ein direktziehender Farbstoff der Formel II 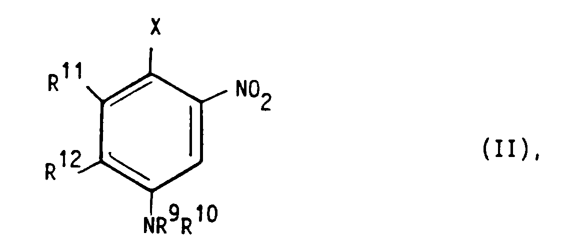 worin R⁹ und R¹⁰ unabhängig voneinander Wasserstoff, C₁-C₄-Alkylgruppen oder Hydroxy- oder Dihydroxy-C₂-C₄-alkylgruppen bedeuten,
R¹¹ und R¹² Wasserstoff, Chlor oder Hydroxy darstellen, mit der Maßgabe, daß R¹¹ Chlor ist, wenn R¹² Wasserstoff oder Hydroxy ist und daß R¹¹ Wasserstoff oder Hydroxy ist, wenn R¹² Chlor ist, und
X Wasserstoff oder eine Gruppe NR¹³R¹⁴ darstellt, wobei R¹³ und R¹⁴ unabhängig voneinander Wasserstoff, C₁-C₄-Alkylgruppen oder Hydroxy- oder Dihydroxy-C₂-C₄-alkylgruppen darstellen
oder
ein direktziehender Farbstoff der Formel III 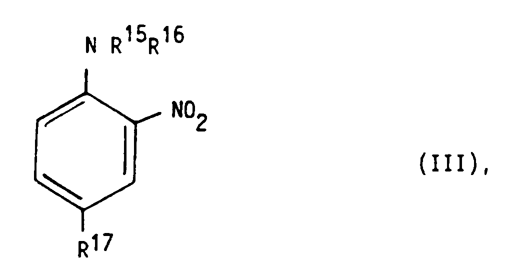 worin R¹⁵ und R¹⁶ unabhängig voneinander Wasserstoff, C₁-C₄-Alkylgruppen oder Hydroxy- oder Dihydroxy-C₂-C₄-alkylgruppen bedeuten und R¹⁷ eine NH₂-Gruppe, eine C₁-C₄-Alkylgruppe oder eine Hydroxy- oder Dihydroxy-C₂-C₄-alkylgruppe darstellt,
enthalten ist.

2. Mittel zum Färben von keratinhaltigen Fasern nach Anspruch 1, dadurch gekennzeichnet, daß die Verbindung der Formel I 2,4,5,6-Tetraaminopyrimidin ist.

3. Mittel zum Färben von keratinhaltigen Fasern nach Anspruch 1 und 2, dadurch gekennzeichnet, daß der Farbstoff der Formel II ausgewählt ist aus 6-Chlor-4-nitro-2-aminophenol, 5-Chlor-4-(2-hydroxyethyl)amino-2-nitroanilin, 5-Chlor-4-(2,3-dihydroxypropyl)amino-2-nitroanilin und 1,4-Bis-[(2,3-dihydroxypropyl)-amino]-5-chlor-2-nitrobenzol und der Farbstoff der Formel III 1-(2-Hydroxyethyl)amino-4-methyl-2-nitrobenzol ist.

4. Haarfärbemittel enthaltend
- 2,4,5,6-Tetraaminopyrimidin in einer Menge von 0,01 bis 5 Gew.-%, vorzugsweise 0,1 bis 1 Gew.-%,
- übliche Kupplerkomponenten in einer Menge von 0,01 bis 5 Gew.-%, vorzugsweise 0,1 bis 1 Gew.-%,
- mindestens einen direktziehenden Farbstoff ausgewählt aus der Gruppe 6-Chlor-4-nitro-2-aminophenol, 5-Chlor-4-(2-hydroxyethyl)amino-2-nitroanilin, 5-Chlor-4-(2,3-dihydroxypropyl)amino-2-nitroanilin und 1,4-Bis-[(2,3-dihydroxypropyl)amino]-5-chlor-2-nitrobenzol in einer Gesamtmenge von 0,005 bis 1 Gew.-%, vorzugsweise 0,01 bis 0,3 Gew.-%,
wobei alle Gew.-% Angaben bezogen sind auf die gesamte Haarfärbemittelzubereitung,
- und einen wasserhaltigen Träger.

5. Haarfärbemittel enthaltend
- 2,4,5,6-Tetraaminopyrimidin in einer Menge von 0,01 bis 5 Gew.-%, vorzugsweise 0,1 bis 1 Gew.-%,
- übliche Kupplerkomponenten in einer Menge von 0,01 bis 5 Gew.-%, vorzugsweise 0,1 bis 1 Gew.-%,
- 1-(2-Hydroxyethyl)amino-4-methyl-2-nitrobenzol in einer Menge von 0,005 bis 1 Gew.-%, vorzugsweise 0,01 bis 0,3 Gew.-%,
wobei alle Gew.-% Angaben bezogen sind auf die gesamte Haarfärbemittelzubereitung,
- und einen wasserhaltigen Träger.

## Claims

1. Formulations for colouring keratin-containing fibers in which at least one 2,4,5,6-tetraaminopyrimidine corresponding to formula I: 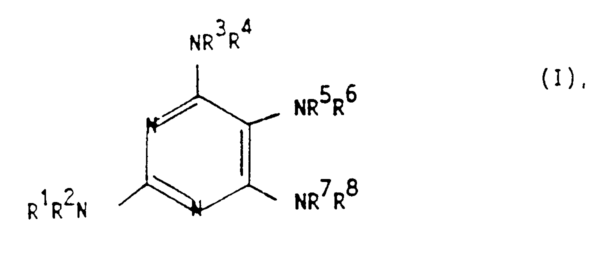 where R¹, R², R³, R⁴, R⁵, R⁶, R⁷ and R⁸ independently of one another represent hydrogen, C₁₋₄ alkyl groups or C₂₋₄ hydroxyalkyl groups,
is present as the primary intermediate together with at least one typical secondary intermediate and a water-containing carrier,
characterized in that a substantive dye corresponding to formula II: 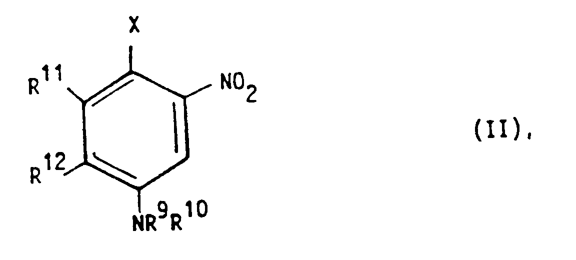 where R⁹ and R¹⁰ independently of one another represent hydrogen, C₁₋₄ alkyl groups or hydroxy or dihydroxy C₂₋₄ alkyl groups, R¹¹ and R¹² represent hydrogen, chlorine or hydroxy, with the proviso that R¹¹ is chlorine where R¹² is hydrogen or hydroxy and R¹¹ is hydrogen or hydroxy where R¹² is chlorine, and X represents hydrogen or a group NR¹³R¹⁴, where R¹³ and R¹⁴ independently of one another represent hydrogen, C₁₋₄ alkyl groups or hydroxy or dihydroxy C₂₋₄ alkyl groups,
or
a substantive dye corresponding to formula III: 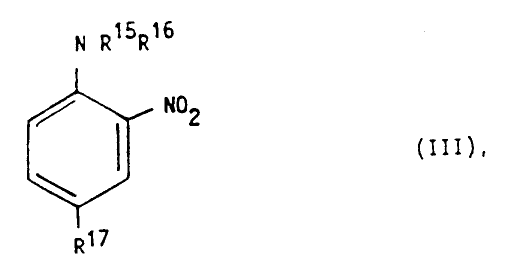 where R¹⁵ and R¹⁶ independently of one another represent hydrogen, C₁₋₄ alkyl groups or hydroxy or dihydroxy C₂₋₄ alkyl groups and R¹⁷ is an NH₂ group, a C₁₋₄ alkyl group or a hydroxy or dihydroxy C₂₋₄ alkyl group,
is present.

2. Formulations for colouring keratin-containing fibres as claimed in claim 1, characterized in that the compound corresponding to formula I is 2,4,5,6-tetraaminopyrimidine.

3. Formulations for colouring keratin-containing fibres as claimed in claims 1 and 2, characterized in that the dye corresponding to formula II is selected from 6-chloro-4-nitro-2-aminophenol, 5-chloro-4-(2-hydroxyethyl)-amino-2-nitroaniline, 5-chloro-4-(2,3-dihydroxypropyl)-amino-2-nitroaniline and 1,4-bis-[(2,3-dihydroxypropyl)-amino]-5-chloro-2-nitrobenzene while the dye corresponding to formula III is 1-(2-hydroxyethyl)-amino-4-methyl-2-nitrobenzene.

4. Hair colouring formulations containing
- 2,4,5,6-tetraaminopyrimidine in a quantity of 0.01 to 5% by weight and preferably in a quantity of 0.1 to 1% by weight,
- typical secondary intermediates in a quantity of 0.01 to 5% by weight and preferably in a quantity of 0.1 to 1 % by weight,
- at least one substantive dye selected from the group consisting of 6-chloro-4-nitro-2-aminophenol, 5-chloro-4-(2-hydroxyethyl)-amino-2-nitroaniline, 5-chloro-4-(2,3-dihydroxypropyl)-amino-2-nitroaniline and 1,4-bis-[(2,3-dihydroxypropyl)-amino]-5-chloro-2-nitrobenzene in a total quantity of 0.005 to 1% by weight and preferably in a total quantity of 0.01 to 0.3% by weight,
all percentages by weight being based on the hair colouring formulation as a whole, and
- a water-containing carrier.

5. Hair colouring formulations containing
- 2,4,5,6-tetraaminopyrimidine in a quantity of 0.01 to 5% by weight and preferably in a quantity of 0.1 to 1% by weight,
- typical secondary intermediates in a quantity of 0.01 to 5% by weight and preferably in a quantity of 0.1 to 1% by weight,
- 1-(2-hydroxyethyl)-amino-4-methyl-2-nitrobenzene in a quantity of 0.005 to 1% by weight and preferably in a quantity of 0.01 to 0.3% by weight,
all percentages by weight being based on the hair colouring formulation as a whole, and
- a water-containing carrier.

## Revendications

1. Agents pour la teinture des fibres kératiniques, renfermant comme composant développateur, au moins
une 2,4,5,6-tétra-aminopyrimidine de la formule I 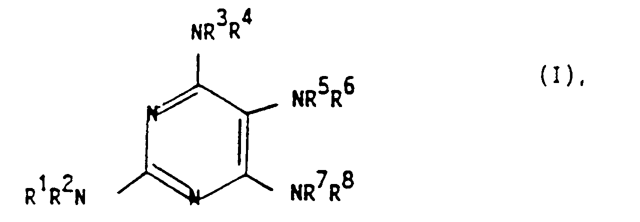 dans laquelle R¹, R², R³, R⁴, R⁵, R⁶, R⁷ et R⁸ représentent, indépendamment les uns des autres, l'hydrogène, un groupe alkyle en C₁-C₄ ou un groupe hydroxyalkyle en C₂-C₄, au moins un composant copulateur usuel et un vecteur aqueux,
caractérisés en ce qu'ils contiennent un colorant montant directement sur la fibre de la formule II, 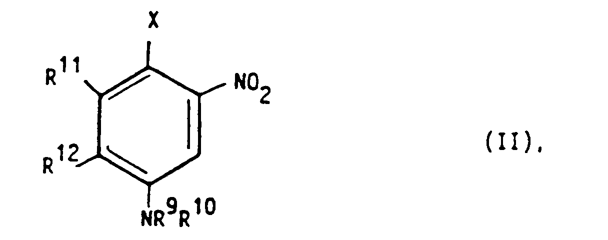 dans laquelle R⁹ et R¹⁰ représentent indépendamment l'un de l'autre l'hydrogène, un groupe alkyle en C₁-C₄ ou un groupe hydroxyalkyle ou dihydroxyalkyle en C₂-C₄, R¹¹ et R¹² correspondent à l'hydrogène, au chlore ou à un hydroxyle, à la condition que R¹¹ soit le chlore si R¹² est l'hydrogène ou un hydroxyle, et que R¹¹ soit l'hydrogène ou un hydroxyle si R¹² est le chlore, et X est l'hydrogène ou un groupe NR¹³R¹⁴, dans lequel R¹³ et R¹⁴ représentent indépendamment l'un de l'autre, l'hydrogène, un groupe alkyle en C₁-C₄ ou un groupe hydroxyalkyle ou dihydroxyalkyle enC₂-C₄,
ou
un colorant montant directement sur la fibre de la formule III 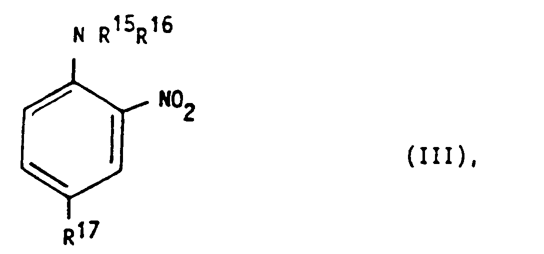 dans laquelle R¹⁵ et R¹⁶ représentent indépendamment l'un de l'autre l'hydrogène, un groupe alkyle en C₁-C₄ ou un groupe hydroxyalkyle ou dihydroxyalkyle enC₂-C₄, et R¹⁷ correspond à un groupe NH₂, à un groupe alkyle en C₁-C₄ ou à un groupe hydroxyalkyle ou dihydroxyalkyle en C₂-C₄.

2. Agents pour la teinture des fibres kératiniques selon la revendication 1, caractérisés en ce que le composé de la formule I est la 2,4,5,6-tétra-aminopyrimidine.

3. Agents pour la teinture des fibres kératiniques selon la revendication 1 ou 2, caractérisés en ce que le colorant de la formule II est sélectionné parmi le groupe du 6-chloro-4-nitro-2-aminophénol, de la 5-chloro-4-(2-hydroxyéthyl)-amino-2-nitroaniline, de la 5-chloro-4-(2,3-dihydroxypropyl)amino-2-nitroaniline et du 1,4-bis-[(2,3-dihydroxypropyl)amino]-5-chloro-2-nitrobenzène, et que le colorant de la formule III est le 1-(2-hydroxyéthyl)amino-4-méthyl-2-nitrobenzène.

4. Teintures capillaires contenant
- de la 2,4,5,6-tétra-aminopyrimidine dans une proportion de 0,01 à 5, de préférence de 0,1 à 1 % en poids,
- des composants copulateurs usuels dans une proportion de 0,01 à 5, de préférence de 0,1 à 1 % en poids,
- au moins un colorant montant directement sur la fibre, sélectionné parmi le groupe du 6-chloro-4-nitro-2-aminophénol, de la 5-chloro-4-(2-hydroxyéthyl)-amino-2-nitroanline, de la 5-chloro-4-(2,3-dihydroxypropyl)amino-2-nitroaniline et du 1,4-bis-[(2,3-dihydroxypropyl)-amino]-5-chloro-2-nitrobenzène, en proportion totale de 0,005 à 1, de préférence de 0,01 à 0,3 % en poids,
toutes les indications en % se rapportant à la totalité de la préparation de teinture capillaire,
- et un vecteur aqueux.

5. Teintures capillaires contenant
- de la 2,4,5,6-tétra-aminopyrimidine dans une proportion de 0,01 à 5, de préférence de 0,1 à 1 % en poids,
- des composants copulateurs usuels dans une proportion de 0,01 à 5, de préférence de 0,1 à 1 % en poids,
- du 1-(2-hydroxyéthyl)amino-4-méthyl-2-nitrobenzène, dans une proportion de 0,005 à 1, de préférence de 0,01 à 0,3 % en poids
toutes les indications en % se rapportant à la totalité de la préparation de teinture capillaire,
- et un vecteur aqueux.
